# EUROPEAN PATENT APPLICATION

(11) **EP 3 967 750 A1**
(43) Date of publication of application: **16.03.2022**
(21) Application number: 20784541.3
(22) Date of filing: 25.03.2020
(51) Int. Cl.: C12N 5/071, C12N 9/74

(54) **THREE-DIMENSIONAL TISSUE CONSTRUCT AND METHOD FOR PRODUCING SAME, AND METHOD FOR PRODUCING CELL-CONTAINING COMPOSITION**

(30) Priority: 01.04.2019 JP 2019069972
(71) Applicant: TOPPAN INC., Tokyo 110-0016 (JP); Osaka University, Suita-shi, Osaka 565-0871 (JP)
(72) Inventor: KITANO Shiro, Tokyo 110-0016 (JP); IRIE Shinji, Tokyo 110-0016 (JP); MATSUSAKI Michiya, Suita-shi, Osaka 565-0871 (JP)
(74) Representative: TBK
(86) International application number: PCT/JP2020/013433
(87) International publication number: WO 2020/203579

(57) **Abstract**

The present invention relates to a method for producing a three-dimensional tissue construct, the method comprising:
a culture step of culturing cells in a culture liquid comprising fragmented extracellular matrix components, fibrin, and an aqueous medium.

## Description

### Technical Field

The present invention relates to a three-dimensional tissue construct and a method for producing the same.

### Background Art

As methods for artificially producing a structure imitating biological tissue, for example, a method (Patent Literature 1) for producing a three-dimensional tissue construct through culturing coated cells in which the entire surface of cultured cells are coated with an adhesive film, a method (Patent Literature 2) for producing a three-dimensional tissue construct, the method including three-dimensionally arranging cells coated with a coating film containing collagen to form the three-dimensional tissue construct, a method (Patent Literature 3) for producing a three-dimensional tissue construct, the method including forming coated cells, in which a coating film is formed on the surface of cells, and arranging the coated cells three-dimensionally, in which the formation of the coated cells includes immersing the cells in a liquid containing a coating component and separating the immersed cells from the liquid containing a coating component using a liquid-permeable film, a method (Patent Literature 4) for producing a three-dimensional cell tissue, the method including mixing cells with a cationic substance and an extracellular matrix component to obtain a mixture and collecting the cells from the obtained mixture to form a cell aggregate on a base material, and the like are known. In addition, the present inventors are proposing a method (Patent Literature 5) for producing a three-dimensional tissue construct having a high collagen concentration by bringing cells into contact with endogenous collagen, and preferably further bringing the cells into contact with fibrous exogenous collagen. It is expected that such three-dimensional tissue constructs will be able to be used as substitutes for laboratory animals, implant materials, or the like.

### Citation List

### Patent Literature

[Patent Literature 1] Japanese Unexamined Patent Publication No. 2012-115254
[Patent Literature 2] PCT International Publication No. WO2015/072164
[Patent Literature 3] PCT International Publication No. WO2016/027853
[Patent Literature 4] PCT International Publication No. WO2017/146124
[Patent Literature 5] PCT International Publication No. WO2018/143286

### Non Patent Literature

[Non Patent Literature 1] K. Holzl et al., Biofabrication 2016, 8, 032002.
[Non Patent Literature 2] Acta Biomaterialia, 2015, Vol. 25, pp. 131-142.

### Summary of Invention

### Technical Problem

Although a three-dimensional tissue construct can be produced according to the above-described methods for producing a three-dimensional tissue construct, the size of a three-dimensional tissue construct that can be produced depends on the size of a culture container. Therefore, it is difficult to control the size, shape, and the like thereof depending on the purpose.

As a method for controlling the shape of cells and placing the cells on a base material, there is a technique called bioprinting (for example, Non Patent Literature 1). However, since bioinks may have too high a viscosity for cells to survive, the cells in a bioink may become considerably damaged.

Therefore, an object of the present invention is to provide a method for easily producing a three-dimensional tissue construct having a controlled shape.

### Solution to Problem

That is, the present invention relates to, for example, each of the following inventions.
[1] A method for producing a three-dimensional tissue construct, the method comprising: a culture step of culturing cells in a culture liquid comprising fragmented extracellular matrix components, fibrin, and an aqueous medium.
[2] The method for producing a three-dimensional tissue construct according to [1], the method further comprising: a step of mixing fibrinogen with thrombin before the culture step.
[3] The method for producing a three-dimensional tissue construct according to [1] or [2], wherein at least some of the fragmented extracellular matrix components are cross-linked.
[4] The method for producing a three-dimensional tissue construct according to any one of [1] to [3], wherein an average length of the fragmented extracellular matrix components is 100 nm to 200 µm.
[5] The method for producing a three-dimensional tissue construct according to any one of [1] to [4], wherein the cells comprise extracellular matrix-producing cells.
[6] The method for producing a three-dimensional tissue construct according to any one of [1] to [5], wherein the cells further comprise one kind or plural kinds of cells selected from the group consisting of vascular endothelial cells, cancer cells, cardiomyocytes, smooth muscle cells, fibroblasts, and epithelial cells.
[7] The method for producing a three-dimensional tissue construct according to any one of [1] to [6], wherein the cells comprise at least vascular endothelial cells and fibroblasts.
[8] The method for producing a three-dimensional tissue construct according to [7], wherein a content of the fibroblasts is greater than or equal to 25% based on the number of whole cells.
[9] The method for producing a three-dimensional tissue construct according to any one of [1] to [8], wherein a content of the fragmented extracellular matrix components is 0.33 mass% to 90 mass% based on the total mass of the fragmented extracellular matrix components and the cells.
[10] The method for producing a three-dimensional tissue construct according to any one of [1] to [9], wherein a content of the fragmented extracellular matrix components is 0.5 mass% to 90 mass% based on the total mass of the fragmented extracellular matrix components and the cells.
[11] The method for producing a three-dimensional tissue construct according to any one of [1] to [10], wherein the culture liquid is placed while being moved in a substantially horizontal direction.
[12] A method for producing a cell-containing composition, the method comprising: a step of arranging a first liquid droplet comprising at least fragmented extracellular matrix components, fibrin, an aqueous medium, and cells and a second liquid droplet comprising at least an aqueous medium so that the liquid droplets come into contact with each other.
[13] The method for producing a cell-containing composition according to [12], wherein the second liquid droplet is placed on the first liquid droplet.
[14] A three-dimensional tissue construct comprising: fragmented extracellular matrix components; fibrin; and cells.

### Advantageous Effects of Invention

According to the present invention, it is possible to easily produce a three-dimensional tissue construct by controlling the shape of the three-dimensional tissue construct. According to the present invention, it is possible to form a three-dimensional tissue construct with a desired shape while suppressing damage to cells.

According to one aspect of the present invention, it is possible to stably produce a three-dimensional tissue construct and to produce a thicker three-dimensional tissue construct. According to one aspect of the present invention, it is possible to easily open the lumen of a blood vessel. According to one aspect of the present invention, it is possible to control the alignment direction of cells to some extent. In the present invention, since a plurality of liquid droplets are formed by changing the types of cells in an aqueous medium, interaction between the cells can be observed. In addition, since liquid droplets can be formed such that a liquid droplet is superposed on another, a taller three-dimensional tissue construct can be produced.

### Brief Description of Drawings

Fig. 1 is photographs and a graph which show fluorescence observation results of three-dimensional tissue constructs having capillaries produced in Test Example 2.
Fig. 2 is micrographs of three-dimensional tissue constructs having capillaries produced in Test Example 2.
Fig. 3 is a graph showing measurement results of the thickness of three-dimensional tissue constructs produced in Test Example 2.
Fig. 4 is photographs showing fluorescence observation results of three-dimensional tissue constructs having capillaries produced in Test Example 2.
Fig. 5 is graphs showing analysis results of fluorescence observation of three-dimensional tissue constructs having capillaries produced in Test Example 2.
Fig. 6 shows photographs showing fluorescence observation results of a three-dimensional tissue construct having capillaries produced in Test Example 2.
Fig. 7 is photographs showing fluorescence observation results of a three-dimensional tissue construct having capillaries produced in Test Example 2.
Fig. 8 is graphs showing analysis results of fluorescence observation of three-dimensional tissue constructs having capillaries produced in Test Example 2.
Fig. 9 is micrographs of a three-dimensional tissue construct having capillaries produced in Test Example 2.
Fig. 10 is a graph showing measurement results of the thickness of three-dimensional tissue constructs produced in Test Example 2.
Fig. 11 is photographs showing fluorescence observation results of a three-dimensional tissue construct having capillaries produced in Test Example 2.
Fig. 12 is photographs showing fluorescence observation results of a three-dimensional tissue construct having capillaries produced in Test Example 2.
Fig. 13 is views showing observation results of a three-dimensional tissue construct produced through 3D printing in Test Example 3.
Fig. 14 is views showing observation results of a three-dimensional tissue construct produced through 3D printing in Test Example 3.
Fig. 15 is views showing observation results of a three-dimensional tissue construct produced through 3D printing in Test Example 3.
Fig. 16 is a graph and photographs which show results of confirming effects of NHDF in Test Example 4.
Fig. 17 is a photograph showing a fluorescence observation result of a three-dimensional tissue construct having capillaries produced in Test Example 4, and a graph showing a diameter of a lumen of the three-dimensional tissue construct.
Fig. 18 is photographs showing fluorescence observation results of three-dimensional tissue constructs having capillaries produced in Test Example 4.
Fig. 19 is photographs showing fluorescence observation results of three-dimensional tissue constructs having capillaries produced in Test Example 5.
Fig. 20 is graphs showing analysis results of fluorescence observation of three-dimensional tissue constructs having capillaries produced in Test Example 5.
Fig. 21 is photographs and a graph which show fluorescence observation results of three-dimensional tissue constructs having capillaries produced in Test Example 6.
Fig. 22 is photographs showing evaluation results of a stability test of Test Example 7.
Fig. 23 is micrographs showing observation results of behavior of a cell population in Test Example 8.
Fig. 24 is micrographs showing observation results of behavior of a cell population in Test Example 8.
Fig. 25 is micrographs showing observation results of behavior of a cell population in Test Example 8.

### Description of Embodiments

Hereinafter, embodiments of the present invention will be described in detail. However, the present invention is not limited to the following embodiments.

### <Method for Producing Three-Dimensional Tissue Construct>

A method for producing a three-dimensional tissue construct according to the present embodiment includes a culture step of culturing cells in a culture liquid containing fragmented extracellular matrix components, fibrin, and an aqueous medium.

### (Three-Dimensional Tissue Construct)

In the present specification, the "three-dimensional tissue construct" means an aggregate of cells in which the cells are three-dimensionally arranged through collagen components and which are artificially produced through cell culture. The shape of the three-dimensional tissue construct is not particularly limited, and examples thereof include a sheet shape, a spherical shape, an ellipsoidal shape, and a rectangular parallelepiped shape. Here, biological tissue includes blood vessels, sweat glands, lymph vessels, sebaceous glands, and the like and has a more complicated structure than the three-dimensional tissue construct. For this reason, it is possible to easily distinguish the three-dimensional tissue construct from biological tissue.

Extracellular matrix components are materials that fill gaps between at least some cells in a three-dimensional tissue construct. The extracellular matrix components are extracellular matrix molecule aggregates formed by a plurality of extracellular matrix molecules. Extracellular matrix molecules may be substances existing outside cells in an organism. Arbitrary substances can be used as extracellular matrix molecules as long as these do not adversely affect growth of cells and formation of cell aggregates. Examples of extracellular matrix molecules include collagen, laminin, fibronectin, vitronectin, elastin, tenascin, entactin, fibrillin, and proteoglycan, but the present invention is not limited thereto. These extracellular matrix components may be used alone or in combination. Extracellular matrix components may, for example, contain collagen or consist of collagen. In a case where extracellular matrix components contain collagen, collagen functions as a foothold for cell adhesion, and formation of a three-dimensional cell structure is further promoted. Extracellular matrix molecules may be modified extracellular matrix molecules or variants of extracellular matrix molecules or may be polypeptides such as chemically synthesized peptides as long as these do not adversely affect the growth of cells and the formation of cell aggregates. Extracellular matrix molecules may have a repetition of a sequence represented by Gly-X-Y which is characteristic to collagen. Here, Gly represents a glycine residue, and X and Y each independently represent an arbitrary amino acid residue. A plurality of Gly-X-Y's may be the same as or different from each other. If extracellular matrix molecules have a repetition of a sequence represented by Gly-X-Y, the degree of binding to a molecular chain arrangement is small and the function as a foothold material is further improved. In the extracellular matrix molecules having the repetition of the sequence represented by Gly-X-Y, the proportion of the sequence represented by Gly-X-Y in the whole amino acid sequence may be greater than or equal to 80% and preferably greater than or equal to 95%. In addition, the extracellular matrix molecules may be polypeptides having an RGD sequence. The RGD sequence is a sequence represented by Arg-Gly-Asp (arginine residue-glycine residue-aspartic acid residue). If the extracellular matrix molecules have an RGD sequence, cell adhesion is further promoted and the extracellular matrix molecules are even more suitable as foothold materials. Examples of extracellular matrix molecules having the sequence represented by Gly-X-Y and the RGD sequence include collagen, fibronectin, vitronectin, laminin, and cadherin.

Examples of collagen include fibrous collagen and non-fibrous collagen. Fibrous collagen means collagen that is a main component of collagen fibers, and specific examples thereof include type I collagen, type II collagen, and type III collagen. Examples of non-fibrous collagen include fibrous type IV collagen.

Examples of proteoglycans include, but are not limited to, chondroitin sulfate proteoglycans, heparan sulfate proteoglycans, keratan sulfate proteoglycans, and dermatan sulfate proteoglycans.

An extracellular matrix component may contain at least one selected from the group consisting of collagen, laminin, and fibronectin and preferably contains collagen. Collagen is preferably fibrous collagen and more preferably type I collagen. Commercially available collagen may be used as fibrous collagen, and specific examples thereof include porcine skin-derived type I collagen manufactured by NH Foods Ltd.

An extracellular matrix component may be derived from animals. Examples of animal species from which extracellular matrix components are derived include, but are not limited to, humans, pigs, and cattle. A component derived from one type of animal may be used as an extracellular matrix component, or components derived from plural kinds of animals may be used in combination. The animal species from which extracellular matrix components are derived may be the same as or different from the origin of cells to be three-dimensionally constructed.

Fragmented extracellular matrix components can be obtained by fragmenting the above-described extracellular matrix components. The "fragmentation" means that an aggregate of extracellular matrix molecules is made smaller in size. The fragmentation may be performed under the conditions of cleaving the bond within extracellular matrix molecules or may be performed under the conditions of not cleaving the bond within extracellular matrix molecules. The fragmented extracellular matrix components may include fibrillated extracellular matrix components in which the above-described extracellular matrix components are fibrillated through application of physical force. Fibrillation is an aspect of fragmentation and is performed under the conditions of, for example, not cleaving the bond within extracellular matrix molecules.

The method for fragmenting extracellular matrix components is not particularly limited. As the method for fibrillating extracellular matrix components, extracellular matrix components may be fibrillated through application of physical force with an ultrasonic homogenizer, a stirring homogenizer, a high-pressure homogenizer, and the like, for example. In a case of using a stirring homogenizer, extracellular matrix components may be homogenized as they are or may be homogenized in an aqueous medium such as physiological saline. In addition, millimeter-sized and nanometer-sized fibrillated extracellular matrix components can also be obtained by adjusting the time, the number of times of homogenizing or the like. Fibrillated extracellular matrix components can also be obtained through fibrillation by repeating freezing and thawing.

Fragmented extracellular matrix components may include at least some fibrillated extracellular matrix components. In addition, fragmented extracellular matrix components may consist of only fibrillated extracellular matrix components. That is, fragmented extracellular matrix components may be fibrillated extracellular matrix components. Fibrillated extracellular matrix components preferably include fibrillated collagen components. Fibrillated collagen components preferably maintain a triple helix structure derived from collagen. Fibrillated collagen components may be components partially maintaining a triple helix structure derived from collagen. If fragmented collagen components are dispersed in an aqueous medium, these can be made likely to come into contact with cells in the aqueous medium to promote formation of a three-dimensional tissue construct.

Examples of the shape of fragmented extracellular matrix components include a fibrous shape. The fibrous shape means a shape composed of a filamentous collagen component or a shape composed of a filamentous extracellular matrix component cross-linked between molecules. At least some fragmented extracellular matrix components may be fibrous. Fibrous extracellular matrix components include, for example, fine filamentous materials (fine fibers) formed by aggregating a plurality of filamentous extracellular matrix molecules, a filamentous material formed by further aggregating fine fibers, and one obtained by fibrillating these filamentous materials. In fibrous extracellular matrix components, since an RGD sequence is preserved without disruption, the fibrous extracellular matrix components can even more effectively function as foothold materials for bonding cells.

The average length of fragmented extracellular matrix components may be 100 nm to 400 µm and 100 nm to 200 µm. In one embodiment, the average length of fragmented extracellular matrix components may be 5 µm to 400 µm, 10 µm to 400 µm, 22 µm to 400 µm, or 100 µm to 400 µm from the viewpoint of facilitating formation of thick tissue. In another embodiment, the average length of fragmented extracellular matrix components may be less than or equal to 100 µm, less than or equal to 50 µm, less than or equal to 30 µm, less than or equal to 15 µm, less than or equal to 10 µm, less than or equal to 1 µm, or greater than or equal to 100 nm from the viewpoints of facilitating stable tissue formation and further improving redispersibility. Of all the fragmented extracellular matrix components, the average length of most of the fragmented extracellular matrix components is preferably within the above-described numerical ranges. Specifically, the average length of 95% of the fragmented extracellular matrix components of all the fragmented extracellular matrix components is preferably within the above-described numerical ranges. The fragmented extracellular matrix components are preferably fragmented collagen components having an average length within the above-described ranges and are more preferably fibrillated collagen components having an average length within the above-described ranges.

The average diameter of fragmented collagen components may be 50 nm to 30 µm, 4 µm to 30 µm, or 5 µm to 30 µm. The fragmented extracellular matrix components are preferably fragmented collagen components having an average diameter within the above-described ranges and are more preferably fibrillated collagen components having an average diameter within the above-described ranges.

The above-described ranges of the average length and the average diameter are optimized from the viewpoint of tissue formation. Therefore, it is desirable that the average length and the average diameter fall within the above-described ranges at a stage where the fragmented extracellular matrix components are used for the tissue formation.

The average length and the average diameter of fragmented extracellular matrix components can be obtained by measuring each fragmented extracellular matrix component using an optical microscope and performing image analysis. In the present specification, the "average length" means an average value of the lengths of the measured samples in the longitudinal direction and the "average diameter" means an average value of the lengths of the measured samples in the direction orthogonal to the longitudinal direction.

At least some fragmented extracellular matrix components may be cross-linked between or within molecules. Fragmented extracellular matrix components may be cross-linked within molecules constituting the fragmented extracellular matrix components or may be cross-linked between molecules constituting the fragmented extracellular matrix components.

Examples of cross-linking methods include a physical cross-linking method through application of heat, ultraviolet rays, radiation, or the like and a chemical cross-linking method using a cross-linking agent or through an enzymatic reaction or the like, but the methods thereof are not particularly limited. Cross-linking (physical cross-linking and chemical cross-linking) may be performed through a covalent bond.

In fragmented collagen components, cross-linking may be formed between collagen molecules (triple helix structure) or may be formed between collagen fibrils formed by collagen molecules. The cross-linking may be cross-linking by heat (thermal cross-linking). Thermal cross-linking can be carried out, for example, by performing heat treatment under reduced pressure using a vacuum pump. In a case of cross-linking collagen molecules, fibrillated collagen components may be cross-linked such that amino groups of collagen molecules form peptide bonds (-NH-CO-) with carboxyl groups of the same or other collagen molecules.

Fragmented extracellular matrix components can also be cross-linked using a cross-linking agent. The cross-linking agent may be one capable of cross-linking carboxyl groups with amino groups or one capable of cross-linking amino groups. Aldehyde-based, carbodiimide-based, epoxide-based, and imidazole-based cross-linking agents are preferable as cross-linking agents from the viewpoints of economical efficiency, safety, and operability, and specific examples thereof include water-soluble carbodiimides such as glutaraldehyde, 1-ethyl-3-(3-dimethylaminopropyl)carbodiimide hydrochloride and 1-cyclohexyl-3-(2-morpholinyl-4-ethyl)carbodiimide sulfonate.

The quantitative determination of the degree of cross-linking can be appropriately selected depending on the types of fragmented extracellular matrix components, means for cross-linking fragmented extracellular matrix components, and the like. The degree of cross-linking may be greater than or equal to 1%, greater than or equal to 2%, greater than or equal to 4%, greater than or equal to 8%, or greater than or equal to 12%, and less than or equal to 30%, less than or equal to 20%, or less than or equal to 15%.

In a case where amino groups in extracellular matrix molecules are used for cross-linking, the degree of cross-linking can be quantitatively determined based on a TNBS method disclosed in Non Patent Literature 2 or the like. The degree of cross-linking obtained through the TNBS method may be within the above-described ranges. The degree of cross-linking obtained through the TNBS method is a proportion of amino groups used for cross-linking among amino groups in the extracellular matrix.

The degree of cross-linking may be calculated by quantitatively determining carboxyl groups. For example, in a case of extracellular matrix components which are insoluble in water, the degree of cross-linking may be quantitatively determined through a toluidine blue O (TBO) method. The degree of cross-linking obtained through the TBO method may be within the above-described ranges.

The content of fragmented extracellular matrix components in a three-dimensional tissue construct may be, for example, greater than or equal to 0.33 mass%, greater than or equal to 0.5 mass%, or greater than or equal to 5 mass% based on the total mass of the fragmented extracellular matrix components and cells. The content of fragmented extracellular matrix components in a three-dimensional tissue construct may be, for example, less than or equal to 90 mass%, less than or equal to 80 mass%, less than or equal to 70 mass%, less than or equal to 60 mass%, less than or equal to 50 mass%, less than or equal to 40 mass%, less than or equal to 30 mass%, less than or equal to 20 mass%, less than or equal to 10 mass%, less than or equal to 5 mass%, or less than or equal to 1 mass% based on the total mass of the fragmented extracellular matrix components and cells. The content of fragmented extracellular matrix components in a three-dimensional tissue construct may be, for example, 0.33 mass% to 90 mass% or 0.5 mass% to 90 mass% based on the total mass of the fragmented extracellular matrix components and cells.

### (Fibrin)

Fibrin is a component obtained by an action of thrombin on fibrinogen to release A and B chains from the N-terminus of an Aα chain and a Bβ chain. Fibrin is a polymer and is usually insoluble in water. Fibrin is formed by bringing fibrinogen into contact with thrombin.

### [Culture Step]

In a culture step, cells are cultured in a culture liquid containing fragmented extracellular matrix components, fibrin, and an aqueous medium. If a culture liquid contains fragmented extracellular matrix components and fibrin, the viscosity of a culture liquid becomes appropriate. Therefore, cells can be cultured in the culture liquid of which the shape is controlled. The culture of cells may be performed in a culture liquid in the form of liquid droplets.

Cells are not particularly limited, but cells may be derived from animals such as humans, monkeys, dogs, cats, rabbits, pigs, cattle, mice, and rats, for example. The origin of cells is also not particularly limited, but cells may be somatic cells derived from the bones, the muscles, the internal organs, nerves, the brain, the bones, the skin, blood, or the like, or may be reproductive cells. Furthermore, cells may be induced pluripotent stem cells (iPS cells) or embryonic stem cells (ES cells) or may be cultured cells such as primary culture cells, subcultured cells, and cell line cells. Specific examples of cells include nerve cells, dendritic cells, immune cells, vascular endothelial cells (for example, human umbilical vein-derived vascular endothelial cells (HUVEC)), lymphatic endothelial cells, fibroblasts, cancer cells such as colorectal cancer cells (for example, human colorectal cancer cells (HT29)) and hepatoma cells, epithelial cells (for example, human gingival epithelial cells), keratinized cells, cardiomyocytes (for example, human iPS cell-derived cardiomyocytes (iPS-CM)), hepatocytes, islet cells, tissue stem cells, smooth muscle cells (for example, aortic smooth muscle cells (Aorta-SMC)), but the present invention is not limited thereto. The cells may include one kind or plural kinds of cells selected from the group consisting of vascular endothelial cells, cancer cells, cardiomyocytes, smooth muscle cells, fibroblasts, and epithelial cells. The cells may be used alone, or plural kinds of cells may be used in combination. The cells may include at least vascular endothelial cells and fibroblasts.

The cells preferably include extracellular matrix-secreting cells that secrete extracellular matrix molecules. Examples of extracellular matrix-secreting cells include collagen-secreting cells that secrete collagen such as fibrous collagen. Examples of collagen-secreting cells include mesenchymal cells such as fibroblasts, chondrocytes, and osteoblasts, and fibroblasts are preferable. Preferred examples of fibroblasts include human skin-derived fibroblasts (NHDF), human cardiac fibroblasts (NHCF), and human gingival fibroblasts (HGF).

The content of fibroblasts based on the number of whole cells may be greater than or equal to 5%, greater than or equal to 10%, greater than or equal to 15%, greater than or equal to 20%, greater than or equal to 25%, or greater than or equal to 30%, and less than or equal to 100%, less than or equal to 95%, less than or equal to 90%, less than or equal to 80%, or less than or equal to 75%.

If cells containing extracellular matrix-secreting cells (for example, collagen-secreting cells) are used, a more stable three-dimensional tissue construct in which cells are uniformly distributed can be obtained. Although the details of the mechanism by which such a three-dimensional tissue construct is obtained is unclear, it is assumed as follows.

In a method for producing a three-dimensional tissue construct in which a foothold in the related art is used, since target cells are injected into the foothold prepared in advance, it is difficult to uniformly distribute cells in the foothold. In a case where cells include collagen-producing cells, the cells first come into contact with and adhere to the top of an extracellular matrix-containing composition. Thereafter, the cells themselves produce proteins (for example, collagen such as fibrous collagen) constituting extracellular matrix components. The produced proteins act as a cross-linking agent by being brought into contact with and adhere to the top of the extracellular matrix-containing composition, and structuring of the proteins or the like constituting the extracellular matrix components in an environment in which the cells are uniformly present progresses. As a result, a more stable three-dimensional tissue construct in which the cells are uniformly distributed is obtained. However, the above-described assumption does not limit the present invention.

In a case where collagen-producing cells and other cells are used together, the proportion of the number of collagen-producing cells to the proportion of other cells (ratio of collagen-producing cells to other cells) may be 9/1 to 99/1, 50/50 to 80/20, 20/80 to 50/50, or 10/90 to 50/50.

In the culture step, cells containing collagen-producing cells and cells other than the collagen-producing cells may be used. The above-described cells can be used as the collagen-producing cells and cells other than the collagen-producing cells. Various model tissues can be produced by producing a three-dimensional tissue construct using the collagen-producing cells and cells other than the collagen-producing cells together. For example, in a case where NHCF and HUVEC are used, it is possible to obtain a three-dimensional tissue construct having capillaries therein. In a case where NHCF and colorectal cancer cells are used, it is possible to obtain a model tissue for colorectal cancer. In addition, in a case where NHCF and iPS-CM are used, it is possible to obtain a model tissue for myocardium showing palpitations.

The method for culturing cells is not particularly limited, and a suitable culture method can be performed depending on the types of cells to be cultured. For example, the culture temperature may be 20°C to 40°C or may be 30°C to 37°C. The pH of a medium may be 6 to 8 or may be 7.2 to 7.4. The culture time may be 1 day to 14 days, 7 days to 14 days, 14 days to 30 days, 30 days to 60 days, or 60 days to 90 days.

A culture liquid may be placed while being moved in, for example, a substantially horizontal direction. If a culture liquid is moved in a substantially horizontal direction, the arrangement of cells, extracellular matrixes, and the like in the culture liquid can be controlled to some extent. If the culture liquid is placed while being moved in a substantially horizontal direction, it is possible to control the tissue structure inside a three-dimensional tissue construct.

The cell density in the culture liquid can be appropriately determined depending on the shape, the thickness, and the like of a target three-dimensional tissue construct. For example, the cell density in the culture liquid may be 1 to 10⁸ cells/mL, or may be 10³ to 10⁷ cells/mL. For example, the cell density in the culture liquid may be the same as that in an aqueous medium in a mixing step to be described below.

The "aqueous medium" means a liquid having water as an essential component. The aqueous medium is not particularly limited as long as extracellular matrix components can be stably present. Examples of aqueous media include physiological saline such as phosphate-buffered physiological saline (PBS) and liquid media such as a Dulbecco's Modified Eagle medium (DMEM) and a vascular endothelial cell-exclusive medium (EGM2), but the present invention is not limited thereto.

The pH of an aqueous medium is preferably within a range that does not adversely affect growth of cells and formation of cell aggregates. The pH of an aqueous medium may be, for example, greater than or equal to 7.0 and less than or equal to 8.0 from the viewpoint of reducing the load on cells when the aqueous medium is placed in the cells. Specifically, the pH of an aqueous medium may be 7.0, 7.1, 7.2, 7.3, 7.4, 7.5, 7.6, 7.7, 7.8, 7.9, or 8.0. An aqueous medium preferably has a buffering capacity within the above-described pH ranges, and is more preferably a liquid medium. The liquid medium is not particularly limited, and a suitable medium can be selected depending on the types of cells to be cultured. Examples of the medium include an Eagle's MEM medium, DMEM, a Modified Eagle medium (MEM), a Minimum Essential medium, RPMI, and a GlutaMax medium. The medium may be a medium to which serum is added, or may be a serum-free medium. Furthermore, the liquid medium may be a mixed medium obtained by mixing two or more kinds of media with each other.

### [Other Steps]

The method for producing a three-dimensional tissue construct according to the present embodiment may include a step of preparing a culture liquid containing fragmented extracellular matrix components, fibrin, an aqueous medium, and cells before the culture step. Fibrin may be formed, for example, by mixing fibrinogen with thrombin.

The method for producing a three-dimensional tissue construct may include a step (mixing step) of mixing cells, fibrinogen, and thrombin with each other in an aqueous medium as a preparatory step before the culture step. Fragmented extracellular matrix components and cells are preferably mixed with fibrinogen and/or thrombin before fibrin is formed by mixing fibrinogen with thrombin.

The mixing step may be a step of, for example, mixing a first liquid containing fibrinogen with a second liquid containing thrombin. Fragmented extracellular matrix components may be contained in the first liquid and/or the second liquid, and cells may be contained in the first liquid and/or the second liquid. The first liquid and the second liquid contain an aqueous medium. Aqueous media in the first liquid and the second liquid may be of the same type or different types.

The mixing step may be carried out such that, for example, a mixture 1 containing cells and fragmented extracellular matrix components is prepared, a mixture 2 obtained by the mixture 1 with fibrinogen is prepared, and the mixture 2 is mixed with thrombin.

In addition, for example, the mixing step may include a step of mixing fibrinogen with fragmented extracellular matrix components to prepare a first mixed liquid, a step of mixing cells with thrombin to prepare a second mixed liquid, and a step of mixing the first mixed liquid with the second mixed liquid. Mixing of components in the mixing step may be performed in an aqueous medium.

In the mixing step, the concentration of fragmented extracellular matrix components can be appropriately determined depending on the shape, the thickness, and the like of a target three-dimensional tissue construct. For example, the concentration of fragmented extracellular matrix components in an aqueous media may be 0.1 to 90 mass% or may be 1 to 30 mass%.

In the mixing step, the amount of fragmented extracellular matrix components may be 0.1 to 100 mg or may be 1 to 50 mg with respect to 1 × 10⁵ cells.

In the mixing step, the mass ratio of fragmented extracellular matrix components to cells is preferably 1/1 to 1,000/1, more preferably 9/1 to 900/1, and still more preferably 10/1 to 500/1.

After the mixing step, gelation of a culture liquid containing cells, fragmented extracellular matrix components, and fibrin may be included before the culture step. The gelation may be performed by, for example, holding the culture liquid under the conditions of 37°C for several tens of minutes to several hours, 1 to 5 hours, or 1.5 to 2.5 hours. The holding time in the gelation step is characterized to be shorter than that in the above-described culture step.

### <Three-Dimensional Tissue Construct>

The three-dimensional tissue construct according to the present embodiment contains cells, fragmented extracellular matrix components, and fibrin. At least some cells may come into contact with the fragmented extracellular matrix components. An aspect of the contact may include adhesion.

In a case where the three-dimensional tissue construct contains extracellular matrix-secreting cells as cells, the three-dimensional tissue construct may contain an endogenous extracellular matrix. The "endogenous extracellular matrix" means an extracellular matrix produced by extracellular matrix-producing cells constituting a three-dimensional tissue construct.

In a case where the three-dimensional tissue construct contains collagen-secreting cells as cells, the three-dimensional tissue construct may contain an endogenous collagen component. The "endogenous collagen" means a collagen component produced by collagen-producing cells constituting a three-dimensional tissue construct. The endogenous collagen may be fibrous collagen or non-fibrous collagen. Since a fragmented collagen component is a collagen component other than a collagen component produced by collagen-producing cells constituting a three-dimensional tissue construct, it can be called an exogenous collagen component.

In a case where a three-dimensional tissue construct contains extracellular matrix-secreting cells as cells, the three-dimensional tissue construct may contain cells containing extracellular matrix-secreting cells, fragmented extracellular matrix components, an endogenous extracellular matrix component, and fibrin. In this case, at least some cells containing extracellular matrix-secreting cells may adhere to fragmented extracellular matrix components and/or an endogenous extracellular matrix component.

The three-dimensional tissue construct may contain extracellular matrix-secreting cells and cells other than the extracellular matrix-secreting cells as cells. Examples of cells other than the extracellular matrix-producing cells include vascular endothelial cells (for example, human umbilical vein-derived vascular endothelial cells (HUVEC)), cancer cells such as colorectal cancer cells (for example, human colorectal cancer cells (HT29)) and hepatoma cells, cardiomyocytes (for example, human iPS cell-derived cardiomyocytes (iPS-CM)), epithelial cells (for example, human gingival epithelial cells), keratinized cells, lymphatic endothelial cells, nerve cells, hepatocytes, tissue stem cells, embryonic stem cells, induced pluripotent stem cells, adhesive cells (for example, immune cells), and smooth muscle cells (for example, aortic smooth muscle cells (Aorta-SMC)). It is preferable that the above-described cells constituting a three-dimensional tissue construct further contain one or more kinds of cells selected from the group consisting of vascular endothelial cells, cancer cells, cardiomyocytes, smooth muscle cells, and epithelial cells.

The content of fragmented extracellular matrix components in a three-dimensional tissue construct may be, for example, within the above-described ranges based on the total mass of the fragmented extracellular matrix components and cells.

In a case where a three-dimensional tissue construct contains an exogenous collagen component, the content of collagen in the three-dimensional tissue construct based on the above-described three-dimensional tissue construct (dry weight) may be 0.01 to 90 mass%, preferably 10 to 90 mass%, 10 to 80 mass%, 10 to 70 mass%, 10 to 60 mass%, 1 to 50 mass%, and 10 to 50 mass%, and more preferably 10 to 30 mass% and 20 to 30 mass%.

Here, "collagen in the three-dimensional tissue construct" means collagen molecules constituting the three-dimensional tissue construct, and may be derived from an endogenous collagen component or may be derived from a fragmented collagen component (which can also be called an exogenous collagen component). That is, in a case where the three-dimensional tissue construct contains an endogenous collagen component and a fragmented collagen component, the content of collagen constituting the above-described three-dimensional tissue construct means a total amount of the endogenous collagen component and the fragmented collagen component. The above-described content of collagen can be calculated from the volume of an obtained three-dimensional tissue construct and the mass of a decellularized three-dimensional tissue construct.

Examples of the method for quantitatively determining the amount of collagen in a three-dimensional tissue construct include the following method for quantitatively determining hydroxyproline. Hydrochloric acid (HCl) is mixed with a solution in which a three-dimensional tissue construct is dissolved, the mixed solution is incubated at a high temperature for a predetermined time. Then, the temperature is returned to room temperature, and a centrifuged supernatant is diluted to a predetermined concentration to prepare a sample. A hydroxyproline standard solution is treated in the same manner as the sample, and then diluted stepwise to prepare a standard. Each of the sample and the standard is subjected to a predetermined treatment with hydroxyproline assay buffer and a detection reagent, and the absorbance at 570 nm is measured. The amount of collagen components is calculated by comparing the absorbance of the sample with that of the standard. A solution obtained by directly suspending and dissolving a three-dimensional tissue construct in high-concentration hydrochloric acid may be centrifuged, and a supernatant may be collected to be used for quantitative determination of collagen. In addition, the three-dimensional tissue construct to be dissolved may be in a state where it is collected from a culture liquid, or may be dissolved in a state where a liquid component is removed by performing a drying treatment after collection. However, in the case where a three-dimensional tissue construct in a state where it is collected from a culture liquid is dissolved to perform quantitative determination of collagen, it is expected that the measurement value of the weight of the three-dimensional tissue construct will vary due to influence of medium components absorbed by the three-dimensional tissue construct and the remainder of a medium due to a problem of an experimental technique. Therefore, it is preferable to use the weight after drying as a reference from the viewpoint of stably measuring the amount of collagen occupying the construct per weight and unit weight.

More specific examples of the method for quantitatively determining the amount of collagen include the following method.

### (Preparation of Sample)

The total amount of a freeze-dried three-dimensional tissue construct is mixed with 6 mol/L HCl and the mixture is incubated in a heat block at 95°C for 20 hours or longer, and then the temperature is returned to room temperature. After centrifugation at 13,000 g for 10 minutes, a supernatant of the sample solution is collected. After the supernatant is appropriately diluted with 6 mol/L HCl so that measurement results to be described below fall within a range of a calibration curve, 200 µL of the supernatant is diluted with 100 µL of ultrapure water to prepare a sample. 35 µL of the sample is used.

### (Preparation of Standard)

125 µL of a standard solution (1,200 µg/mL in acetic acid) and 125 µL of 12 mol/L HCl are placed in a screw-cap tube and mixed with each other, and the mixture is incubated in a heat block at 95°C for 20 hours. Then, the temperature is returned to room temperature. After centrifugation at 13,000 g for 10 minutes, a supernatant is diluted with ultrapure water to prepare 300 µg/mL S1, and the S1 is diluted stepwise with ultrapure water to prepare S2 (200 µg/mL), S3 (100 µg/mL), S4 (50 µg/mL), S5 (25 µg/mL), S6 (12.5 µg/mL), and S7 (6.25 µg/mL). S8 (0 µg/mL) containing only 90 µL of 4 mol/L HCl is also prepared.

### (Assay)

35 µL of each of the standard and the samples is placed on a plate (attached in QuickZyme Total Collagen Assay Kit, QuickZyme Biosciences). 75 µL of assay buffer (attached in the above-described kit) is added to the wells. The plate is closed with a seal and incubated at room temperature while being shaken for 20 minutes. The seal is removed, and 75 µL of a detection reagent (reagent A:reagent B = 30 µL:45 µL, attached in the above-described kit) is added to the wells. The plate is closed with a seal, and the solutions are mixed with each other through shaking and incubated at 60°C for 60 minutes. The mixture is sufficiently cooled on ice, and the seal is removed to measure the absorbance at 570 nm. The amount of collagen is calculated by comparing the absorbance of the samples with that of the standard.

Collagen components occupying a three-dimensional tissue construct may be defined by an area ratio or a volume ratio thereof. The "definition by an area ratio or a volume ratio thereof' means that the proportion of a region where collagen components occupying the entire three-dimensional tissue construct are present is calculated through naked-eye observation or using various kinds of microscopes, image analysis software, and the like after the collagen components in the three-dimensional tissue construct are made to be distinguishable from other tissue components through, for example, a well-known staining technique (for example, immunostaining in which an anti-collagen antibody is used or Masson's Trichrome staining). In a case where the collagen component is defined by the area ratio thereof, there is no limitation on which a cross section or surface in a three-dimensional tissue construct defines the area ratio. However, in a case where the three-dimensional tissue construct is a spherical body or the like, the collagen component may be defined by a cross-sectional view passing through a substantially central portion thereof.

For example, in a case where the collagen components in a three-dimensional tissue construct are defined by an area ratio, the proportion of the area thereof is preferably 0.01% to 99%, 1% to 99%, 5% to 90%, 7% to 90%, or 20% to 90%, and more preferably 50% to 90% based on the total area of the above-described three-dimensional tissue construct. The "collagen components in a three-dimensional tissue construct" are as described above. The proportion of the area of the collagen components constituting a three-dimensional tissue construct means a proportion of the total area of an endogenous collagen component and an exogenous collagen component. The proportion of the area of the collagen components can be calculated, for example, as a proportion of an area of collagen components stained blue with respect to the total cross-sectional area passing through a substantially central portion of an obtained three-dimensional tissue construct subjected to Masson's Trichrome staining.

A residual rate of a three-dimensional tissue construct subjected to trypsin treatment at a trypsin concentration of 0.25%, a temperature of 37°C, a pH of 7.4, and a reaction time of 15 minutes is preferably greater than or equal to 70%, more preferably greater than or equal to 80%, and still more preferably greater than or equal to 90%. Such a three-dimensional tissue construct is stable because it is unlikely to be decomposed by an enzyme during or after culturing. The above-described residual rate can be calculated from the mass of a three-dimensional tissue construct before and after trypsin treatment, for example.

The residual rate of the above-described three-dimensional tissue construct subjected to collagenase treatment at a collagenase concentration of 0.25%, a temperature of 37°C, a pH of 7.4, and a reaction time of 15 minutes may be greater than or equal to 70%, more preferably greater than or equal to 80%, and still more preferably greater than or equal to 90%. Such a three-dimensional tissue construct is stable because it is unlikely to be decomposed by an enzyme during or after culturing.

The thickness of the above-described three-dimensional tissue construct is preferably greater than or equal to 10 µm, more preferably greater than or equal to 100 µm, and still more preferably greater than or equal to 1,000 µm. Such a three-dimensional tissue construct is a structure closer to biological tissue, and is suitable as a substitute for a laboratory animal and an implant material. The upper limit of the thickness of a three-dimensional tissue construct is not particularly limited, but may be, for example, less than or equal to 10 mm, less than or equal to 3 mm, less than or equal to 2 mm, less than or equal to 1.5 mm, and less than or equal to 1 mm.

Here, the "thickness of a three-dimensional tissue construct" means the distance between both ends in a direction perpendicular to the main surface in a case where the three-dimensional tissue construct has a sheet shape or a rectangular parallelepiped shape. In a case where the above-described main surface is uneven, the thickness means the distance therebetween at the thinnest portion of the above-described main surface.

In addition, in a case where a three-dimensional tissue construct has a spherical shape, the thickness thereof means the diameter thereof. In addition, in a case where a three-dimensional tissue construct has an ellipsoidal shape, the thickness thereof means the minor axis thereof. In a case where a three-dimensional tissue construct has a substantially spherical shape or a substantially ellipsoidal shape and its surface is uneven, the thickness thereof means the shortest distance between two points where a straight line passing through the gravity center of the three-dimensional tissue construct and the above-described surface intersect.

If a culture liquid containing fragmented extracellular matrix components and fibrin is used, cells can be cultured in the culture liquid of which the shape is controlled. That is, according to one embodiment, a cell-containing composition which contains fragmented extracellular matrix components, fibrin, and cells and of which the shape is controlled can be formed. A three-dimensional tissue construct is formed by culturing the cell-containing composition. The shape of a culture liquid (cell-containing composition) can be controlled through, for example, a method such as 3D printing. In a case where the control is carried out through the 3D printing method, the control may be performed using a 3D printer or through a manual operation using a pipette.

In one embodiment of the present invention, there is provided a method for producing a cell-containing composition, the method including: a step (arrangement step) of arranging a first liquid droplet containing at least fragmented extracellular matrix components, fibrin, an aqueous medium, and cells (first cells) and a second liquid droplet containing at least an aqueous medium so that the liquid droplets come into contact with each other. According to the production method, a cell-containing composition that can evaluate the behavior of a cell population (behavior of cells as a population) in the first liquid droplet when the first liquid droplet is brought into contact with the second liquid droplet is provided. According to this cell-containing composition, since it is possible to easily perform image analysis when the behavior of the cell population is observed, more efficient evaluation becomes possible. The method for producing a cell-containing composition may further include a step of culturing cells after the arrangement step. The cell culture conditions or the like are as described above. The cell-containing composition obtained through the method of the present embodiment is suitable for evaluating the behavior of a cell population because the shape of the cell-containing composition is likely to be maintained through a cell culture period.

The second liquid droplet may be placed so that a part of the side surface of the first liquid droplet (for example, a part of the outer circumference of the first liquid droplet) comes into contact therewith, or may be placed on the first liquid droplet. The second liquid droplet may further contain fragmented extracellular matrix components, may further contain fibrin, may further contain a specimen, or may contain fragmented extracellular matrix components, fibrin, and a specimen. The specimen may be a substance that can act on the first cells, or may be cells (second cells) different from the first cells. In a case where the first cells are HUVEC, examples of specimens include NHDF, VEGF, and a combination thereof. One or two or more second liquid droplets may be arranged so as to come into contact with the first liquid droplets.

The method for producing a cell-containing composition which includes the above-described arrangement step can also be regarded as a part of the method for evaluating the behavior of a cell population (behavior of cells as a population). For example, a first liquid droplet containing at least fragmented extracellular matrix components, fibrin, an aqueous medium, and first cells is first prepared. A second liquid droplet at least containing an aqueous medium is placed so as to come into contact with the first liquid droplet. The behavior of the first cells in the first liquid droplet as a population is observed by observing a portion near the interface between the first liquid droplet and the second liquid droplet and a portion in which the second liquid droplet does not come into contact with the first liquid droplet using a microscope or the like.

### Example

Hereinafter, the present invention will describe examples specifically, but is not limited by these examples.

### <Test Example 1: Production of Fibrillated Collagen Components>

100 mg of porcine skin-derived type I collagen freeze-dried body manufactured by NH Foods Ltd. was heated at 200°C for 24 hours in Vacuum Sample Drying Oven HD-15H (manufactured by Ishii Laboratory Works Co., Ltd.) under reduced pressure. Accordingly, collagen components (cross-linked collagen components), at least some of which were cross-linked, were obtained. No significant change in appearance was observed in collagen before and after the heating at 200°C.

50 mg of the produced cross-linked collagen components were suspended in 5 mL of ultrapure water, and the suspension was homogenized with an ultrasonic homogenizer by repeating a cycle of 20 seconds 10 times under the condition of 4°C to fibrillate the cross-linked collagen components. The obtained liquid was filtered with a filter having a pore diameter of 35 µm to obtain a dispersion liquid containing the fibrillated collagen components (sCMF).

The average length of sCMF was 14.8 ± 8.2 µm (N=20).

### <Test Example 2: Production and Evaluation of Three-Dimensional Tissue Construct>

Reagents, instruments, and production conditions used for producing a three-dimensional tissue construct are as follows.
Bovine plasma-derived fibrinogen (Sigma F8630, Type I-S, 65-85%, Protein)
Bovine plasma-derived thrombin (Sigma T4648, 66N1H unit/mg protein)
Total number of cells: 5 × 10⁶ cells/mL (NHDF:HUVEC=2:1)
Media (EBM-2:DMEM (High Glucose)=1:1)
EBM-2: Lonza, Endothelial Cell Basal Medium-2
DMEM (High Glucose): NACALAI TESQUE, INC.
Glass bottom dish (Matsunami Glass Ind., Ltd., 35 mmφ dish, and D1114OH)
Medium: 4 mL (replace every 2 days), 37°C, and 5% CO₂

Fibrinogen was dissolved in serum-free DMEM (NACALAI TESQUE, INC., 08489-45) at a concentration of 20 mg/mL in a constant-temperature tank at 37°C to prepare a fibrinogen solution. Thrombin was dissolved in the DMEM at a concentration of 100 unit/mL in the constant-temperature tank at 37°C to prepare a thrombin solution. sCMF was dispersed in the DMEM at a concentration of 60 mg/mL to prepare a sCMF dispersion liquid.

The cells, 18 µL of DMEM, and 2 µL of a thrombin solution were added to a 1.5 mL sample tube (WATSON, 131-7155C). 40 µL of a mixed liquid obtained by mixing the fibrinogen solution with the sCMF dispersion liquid at a ratio of 1:1 was added to the sample tube in which the cells and thrombin were placed, and the mixture was pipetted to prepare a test solution for forming a construct. 30 µL of the test solution for forming a construct in the sample tube was added dropwise onto a glass bottom dish to form liquid droplets. After the liquid droplets were incubated for 2 hours at 37°C, 3 mL of DMEM was added thereto to start culture. The culture was carried out for 5 to 14 days. Accordingly, a three-dimensional tissue construct having capillaries was produced.

The three-dimensional tissue construct was produced so that the sCMF was 0.33 mass%, 0.5 mass%, 1 mass%, or 2 mass% based on the total mass of the sCMF and the cells. In addition, a comparative construct (sCMF content: 0 mass%) was produced similarly to the above except that no sCMF was used.

Fluorescence imaging of the three-dimensional tissue construct was performed according to the following procedure. The obtained three-dimensional tissue construct was immobilized using 4% paraformaldehyde (PFA) at room temperature (25°C). The immobilized three-dimensional tissue construct was subjected to washing with 1% PBS for 15 minutes three times. Next, the three-dimensional tissue construct was treated with 0.2% Triton-X100 (Sigma) PBS for 15 minutes at room temperature and was subjected to washing with 1% PBS for 15 minutes three times again. The obtained three-dimensional tissue construct was treated with 1% Bovine Serum Albumin (BSA, Sigma, A3294)-PBS for 1 hour at room temperature. The treated three-dimensional tissue construct was allowed to act under the conditions of 4°C for 12 hours using 1 mL of 1% BSA-PBS containing CD31 (monoclonal mouse anti-human CD31 antibody, Endothelial Cells, Clone JC70A, Dako). After repeating the washing with 1% PBS for 15 minutes three times, the obtained three-dimensional tissue construct was allowed to act by diluting Alexa Fluor 647 (Alexa Fluor 647 Goat Anti-Mouse IgG (H+L), Invitrogen) or Alexa Fluor 488 and Hoechst (Hoechst 33342) with 1% BSA-PBS to 1/200 and 1/100, respectively.

A confocal laser microscope (Yokogawa, CQI) was used to observe the stained three-dimensional tissue construct, and laser excitation light was set to 488 nm, 640 nm, and 405 nm. The fluorescence area was analyzed using ImageJ.

The thickness of the three-dimensional tissue construct was measured using EVOS FL Auto Software in Measure Model (N=30). The diameter of the capillaries was measured using ImageJ (N=100).

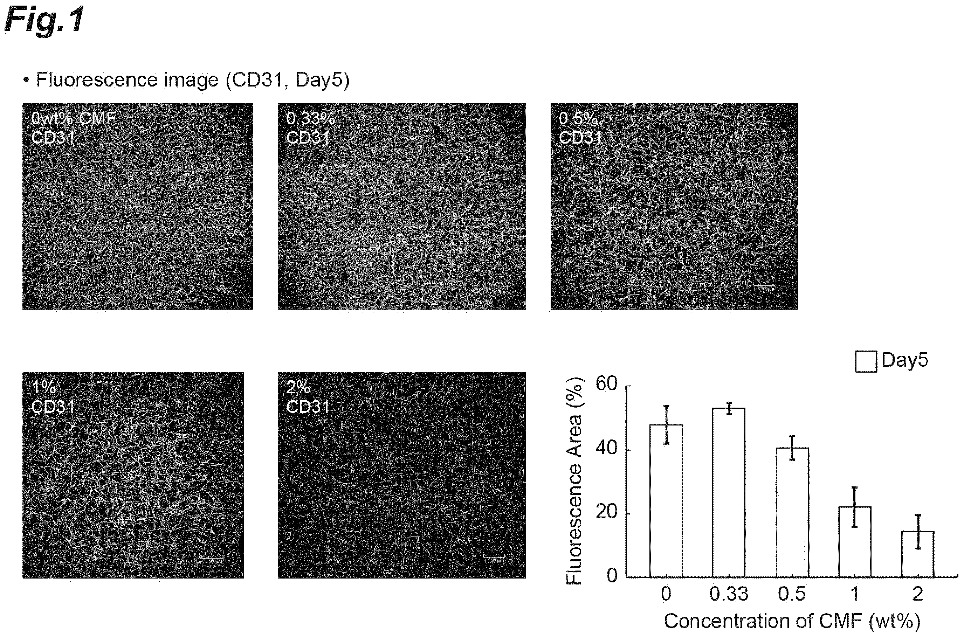

Fig. 1 shows fluorescence observation results of three-dimensional tissue constructs having capillaries. It is shown that the capillaries are formed in a case where the three-dimensional tissue constructs contain sCMF.

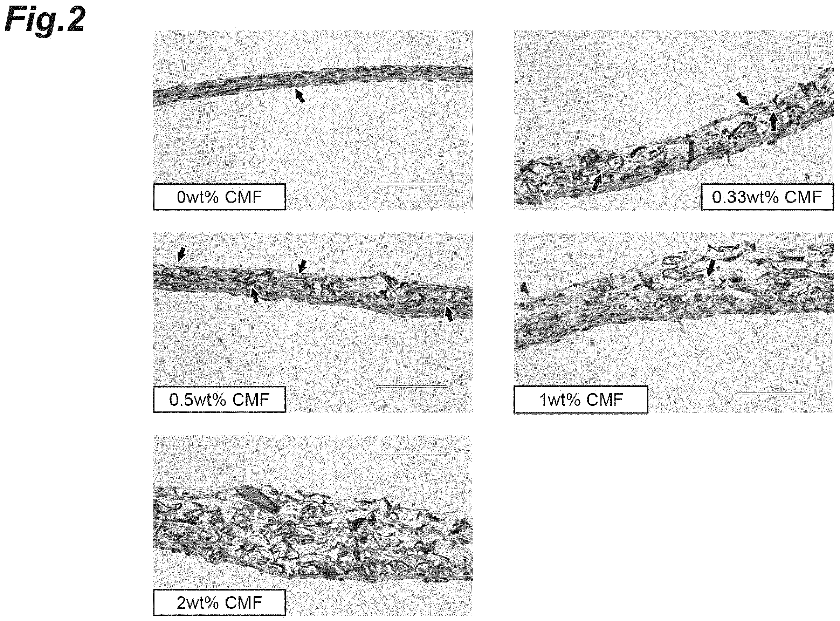

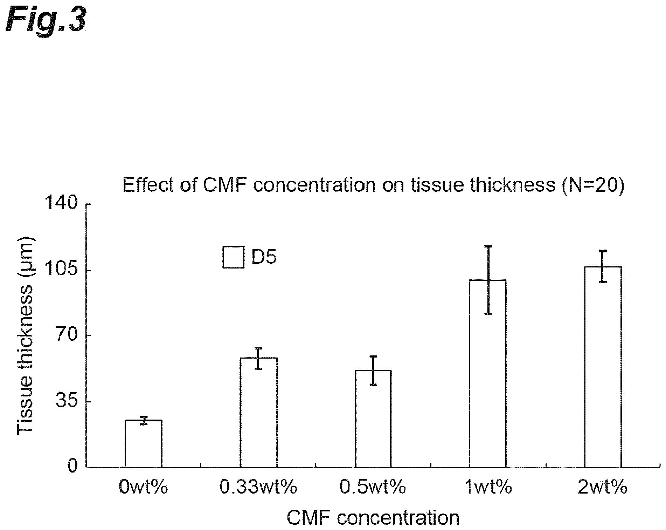

Fig. 2 shows microscopic observation results of three-dimensional tissue constructs produced. The arrows in Fig. 2 indicate the lumens of blood vessels. Fig. 3 is a graph showing a relationship between the thickness of three-dimensional tissue constructs and the content of sCMF. It is shown in Figs. 2 and 3 that thicker three-dimensional tissue constructs can be obtained in the case where three-dimensional tissue constructs contain sCMF.

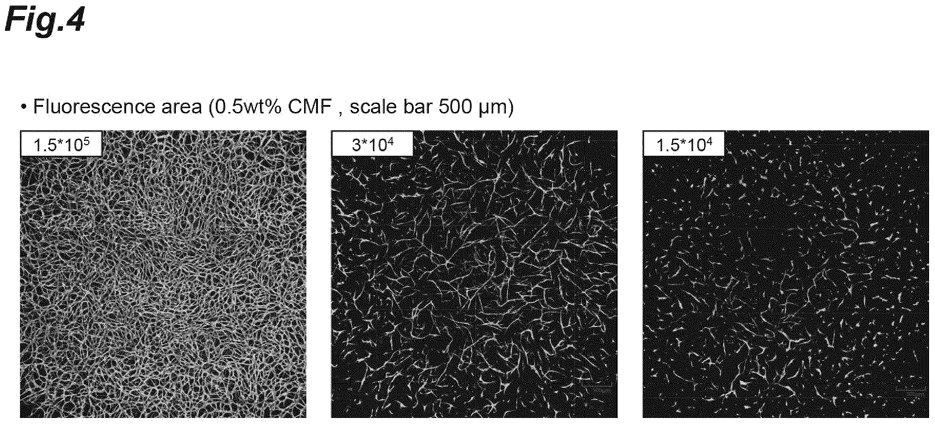

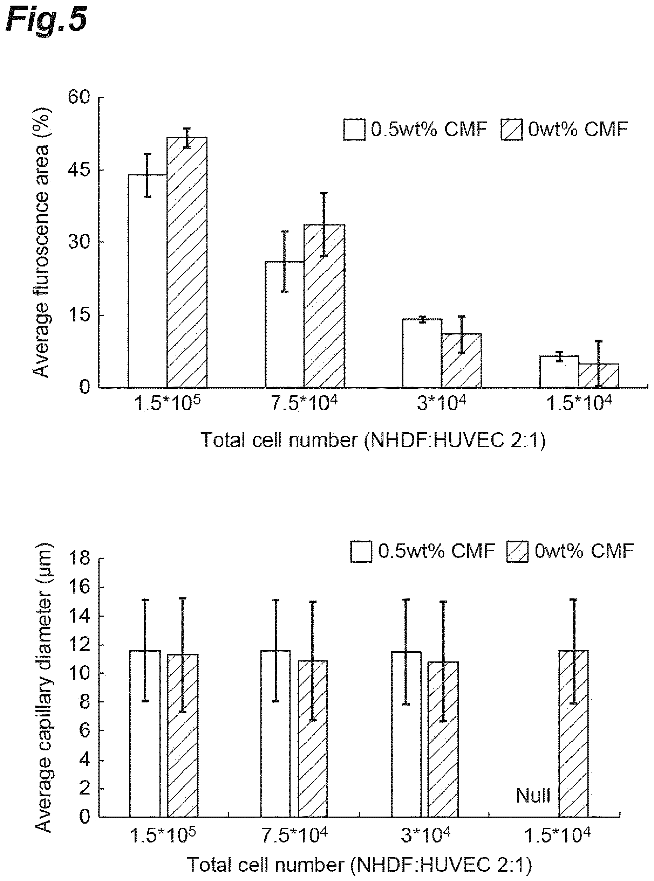

Three-dimensional tissue constructs having capillaries were produced through the above-described method while setting the total number of cells (NHDF:HUVEC=2:1) to 1.5 × 10⁵ cells/mL, 3.0 × 10⁴ cells/mL, or 1.5 × 10⁴ cells/mL. Fig. 4 shows fluorescence observation results of three-dimensional tissue constructs produced under the condition of 0.5 mass% of sCMF. Fig. 5 is graphs showing a relationship between the number of cells and the fluorescence area or the diameter of the lumen. It was confirmed that capillaries are likely to be formed in accordance with increase in the number of cells.

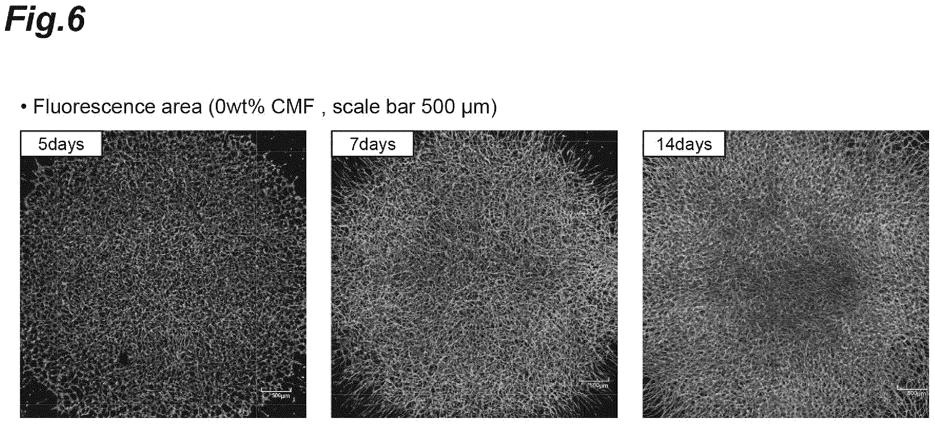

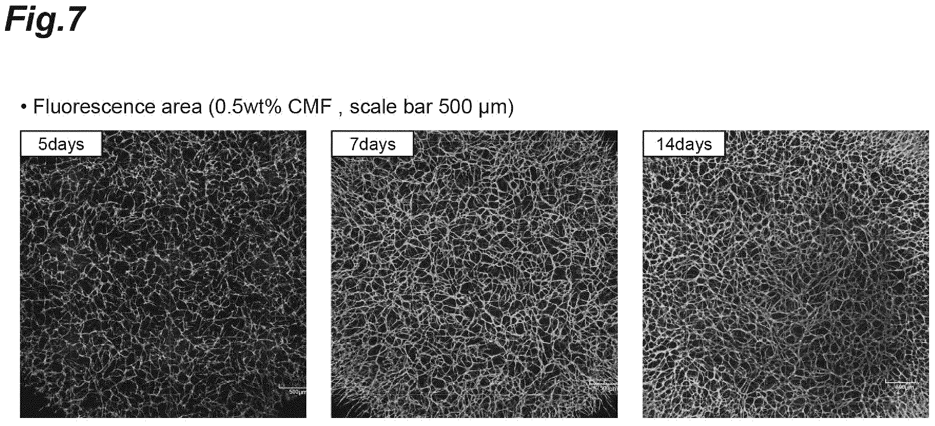

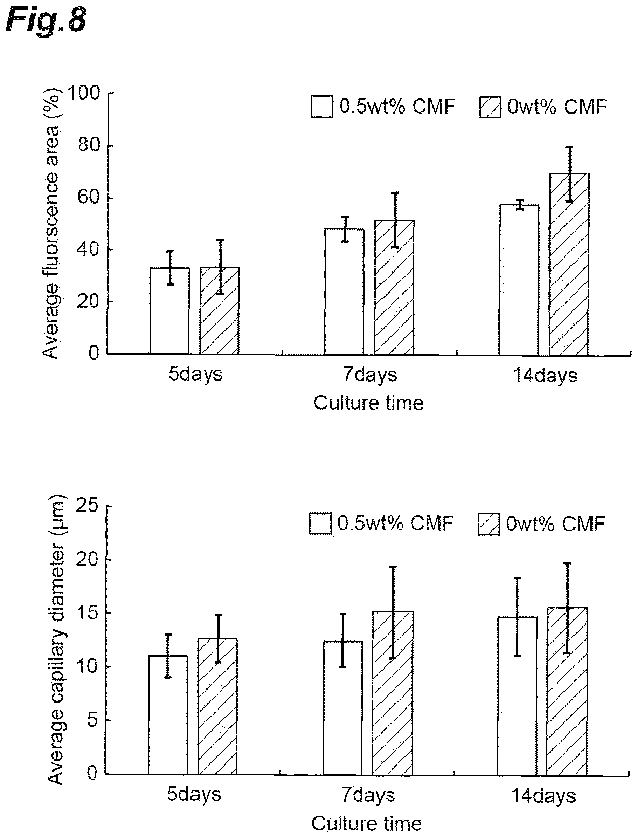

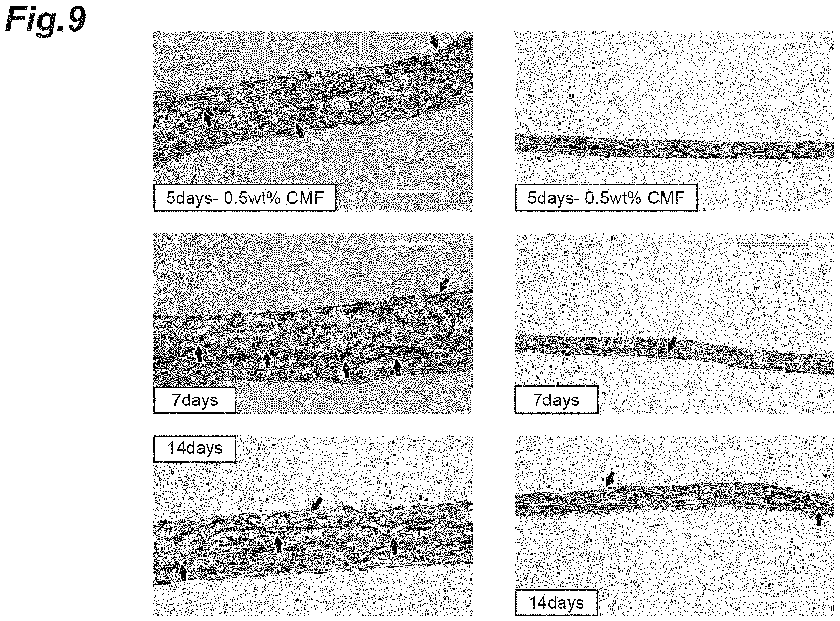

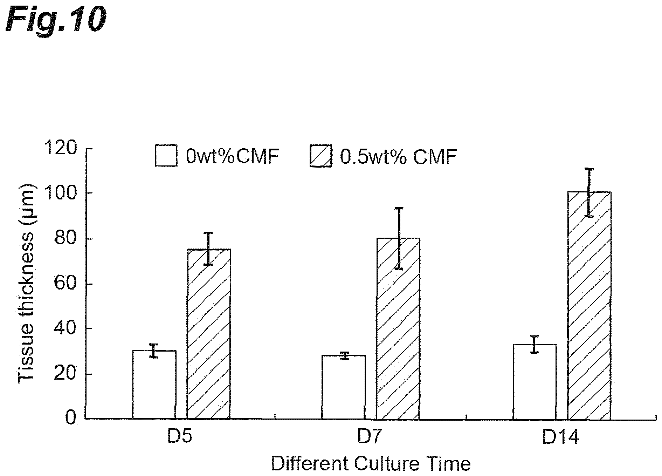

Fluorescence observation results of three-dimensional tissue constructs and comparative constructs having a culture period of 5 days, 7 days, or 14 days are shown in Figs. 6 and 7. Fig. 8 is graphs showing a relationship between the number of days of culture and the fluorescence area or the diameter of the lumen. As shown in Figs. 6 to 8, the fluorescence area and the diameter of the lumens of all of the constructs increased as the culture period became longer. Observation results of three-dimensional tissue constructs having a culture period of 5 days, 7 days, or 14 days are shown in Fig 9. Fig. 10 is a graph showing the thickness of three-dimensional tissue constructs and the comparative constructs. As shown in Fig. 10, it was confirmed that thicker constructs were formed by using sCMF.

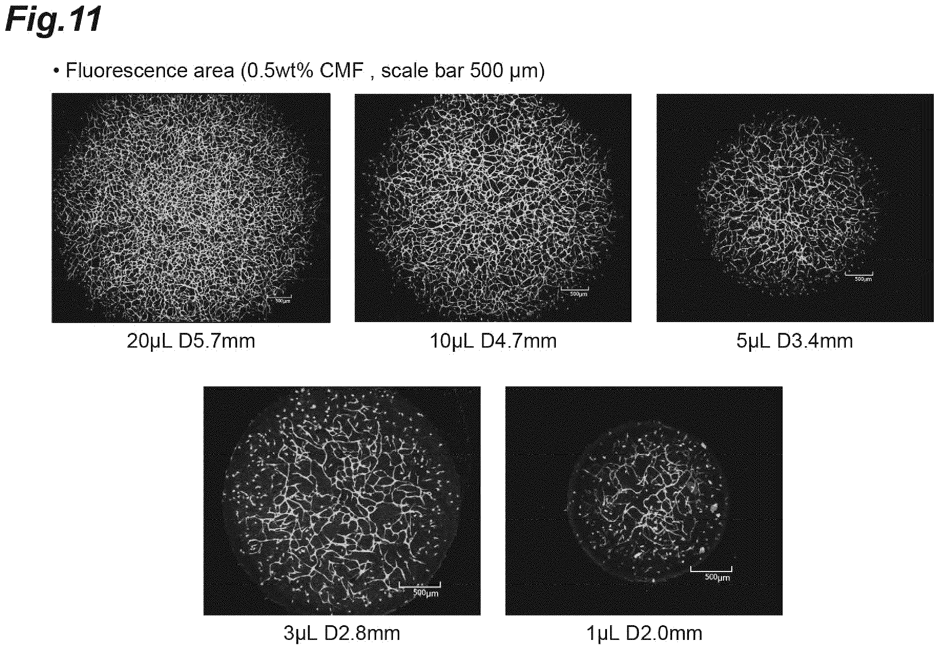

It was shown (in Fig. 11) that the shape of three-dimensional tissue constructs having capillaries can be easily controlled by adjusting the dropping amount of a test solution for forming a construct.

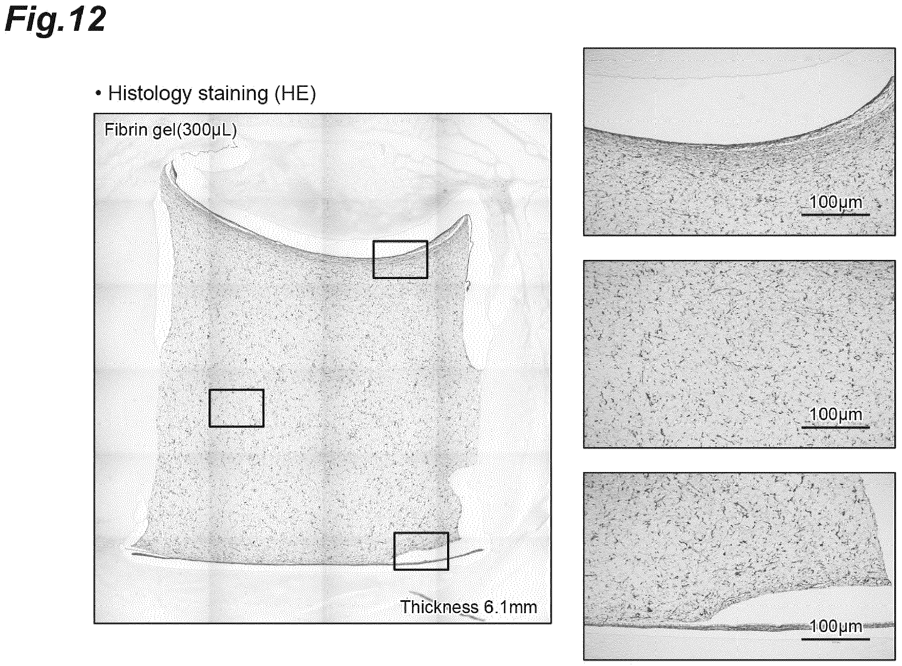

Fig. 12 shows a three-dimensional tissue construct produced by setting the dropping amount of a test solution for forming a construct to 300 µL. The thickness of the three-dimensional tissue construct shown in Fig. 12 was 6.1 mm.

### <Test Example 3: Production and Evaluation of Three-Dimensional Tissue Construct through 3D Printing>

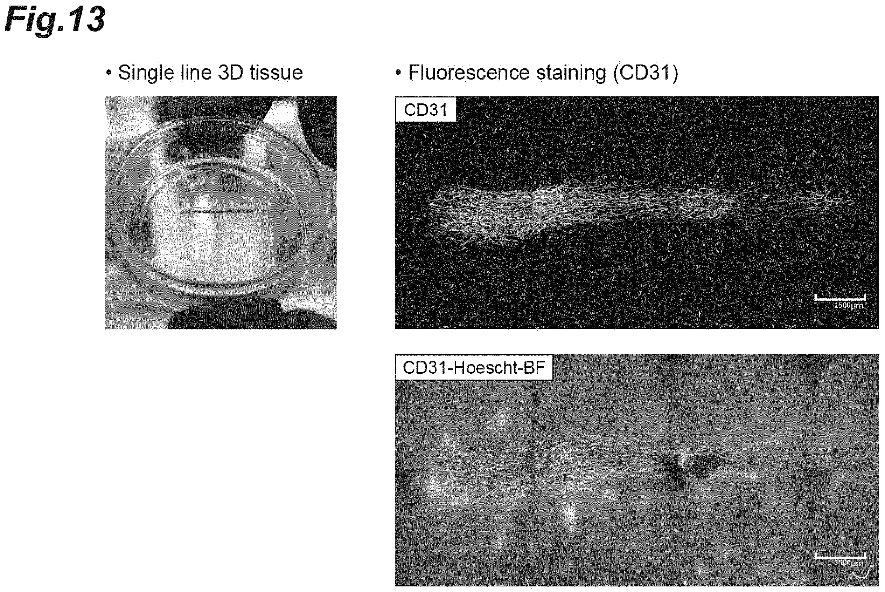

The test solution for forming a construct produced through the above-described method was placed on a dish so as to have a shape (linear shape) shown in Fig. 13 through 3D printing. The conditions for 3D printing at this time are as follows. The results are shown in Fig. 13.
0.5 mass% Fibrinogen
10 unit/mL Thrombin
5 × 10⁶ Cells (NHDF:HUVEC 2:1)
20 to 30 Minutes of gelation time

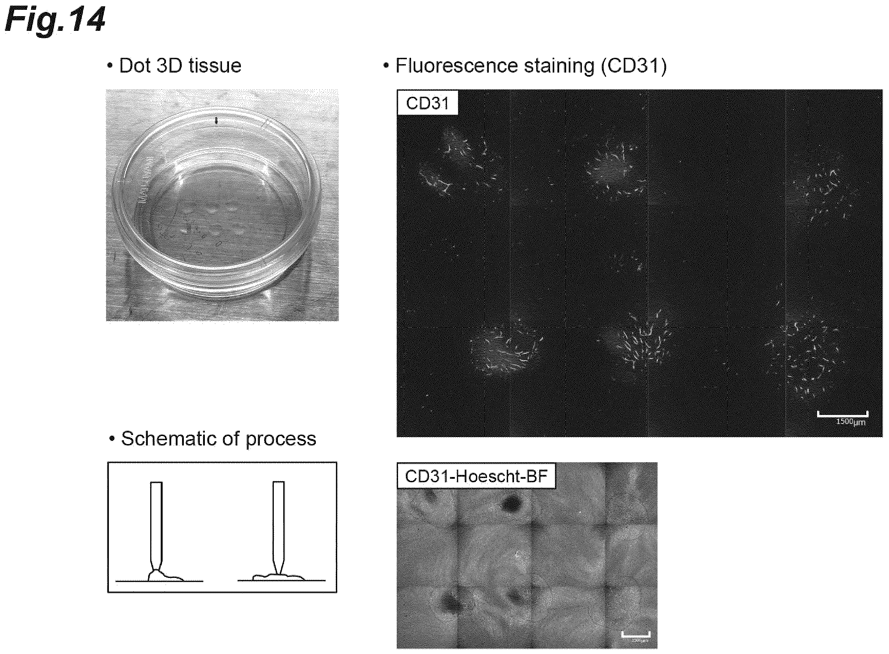

The test solution for forming a construct produced through the above-described method was placed on a dish so as to have a shape shown in Fig. 14 through 3D printing. The test solution was placed on the dish so as to spread. The conditions for 3D printing at this time are as follows. The results are shown in Fig. 14.
0.5 mass% Fibrinogen
10 unit/mL Thrombin
5 × 10⁶ Cells (NHDF:HUVEC 2:1)
20 to 30 Minutes of gelation time

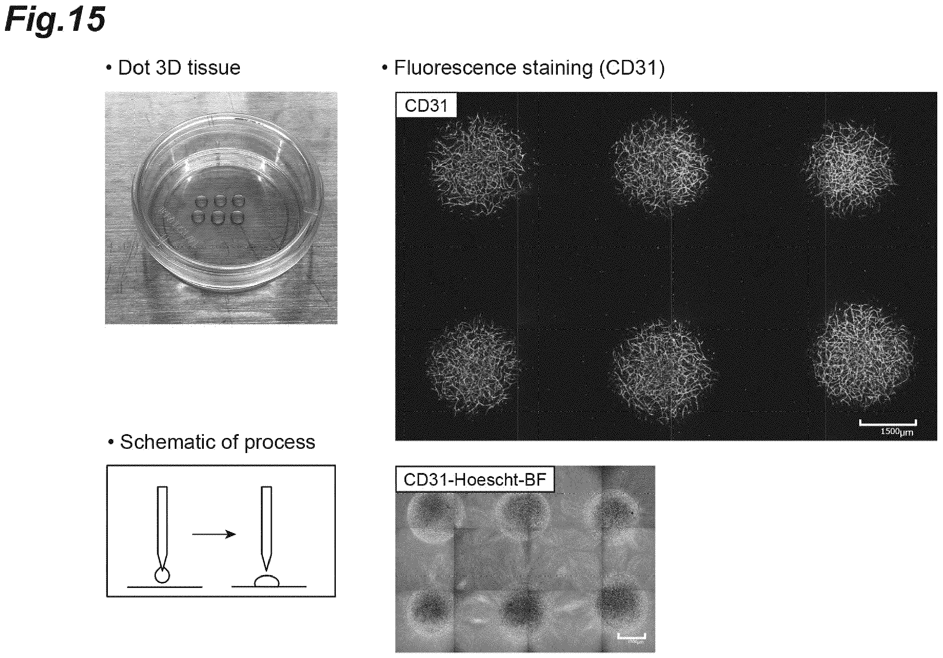

The test solution for forming a construct produced through the above-described method was placed on a dish so as to have a shape (dot shape) shown in Fig. 15 through 3D printing. When placing the test solution on the dish, the test solution was placed thereon through dropwise addition without spreading. The conditions for 3D printing at this time are as follows. The results are shown in Fig. 15.
0.5 mass% Fibrinogen
10 unit/mL Thrombin
5 × 10⁶ Cells (NHDF:HUVEC 2:1)
20 to 30 Minutes of gelation time

As shown in Figs. 13 to 15, it was shown that three-dimensional tissue constructs having desired shapes can be formed. In addition, it was shown that orientation inside a construct to be formed can also be controlled through 3D printing (refer to Fig. 13).

### [Test Example 4: Effect of NHDF]

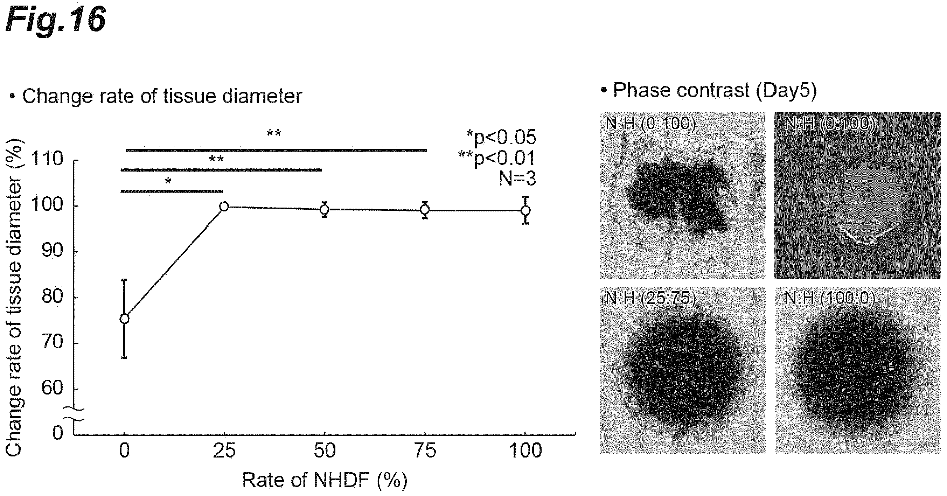

Three-dimensional tissue constructs were produced in the same manner as above except that the ratio of the number of cells of NHDF to HUVEC (NHDF (N) : HUVEC (H)) was set to 0:100, 25:75, 50:50, 75:25, or 100:0. As shown in Fig. 16, it was shown that tissue stability was further improved in a case where the ratio of the number of cells of NHDF to HUVEC is greater than or equal to 25%.

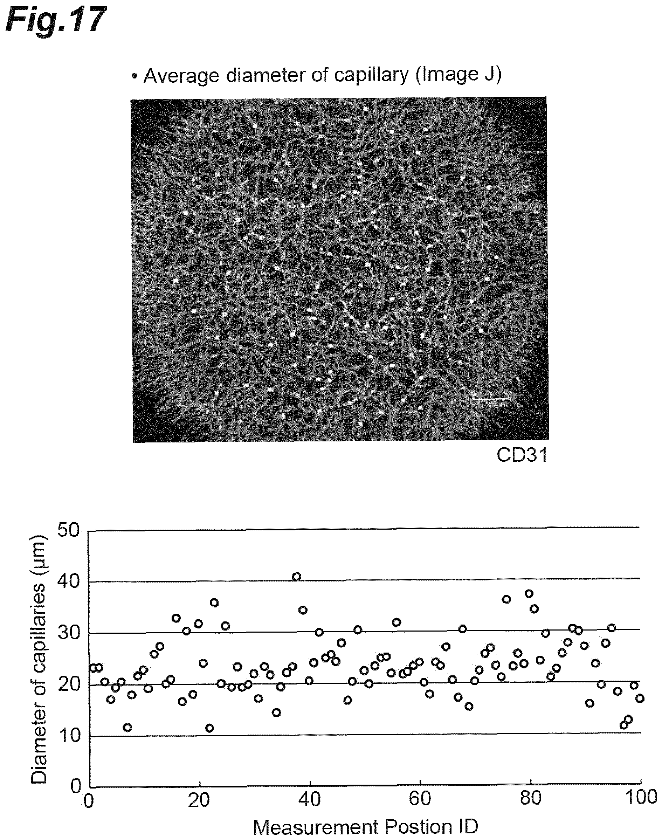

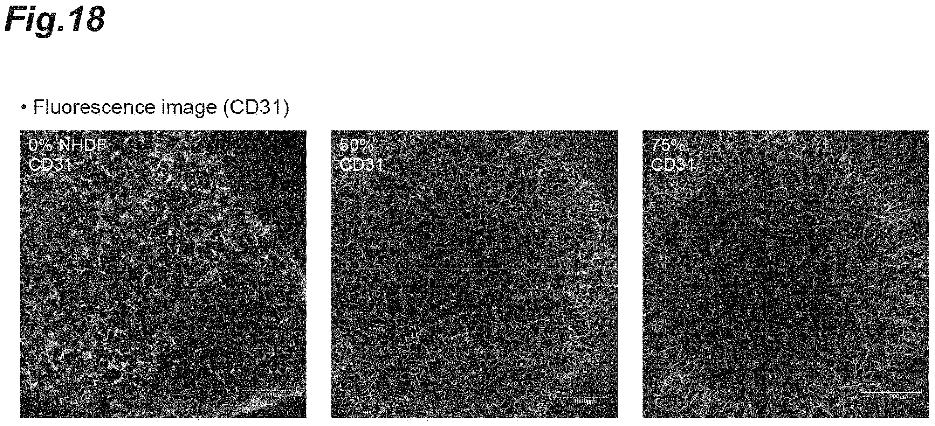

A photograph showing a fluorescence observation result of the produced three-dimensional tissue construct having capillaries and measurement results of the diameter of the lumens in which the ratio of the number of cells of NHDF to HUVEC is set to 25:75 are shown in Fig. 17. Fig. 18 is photographs showing fluorescence observation results of produced three-dimensional tissue constructs in which the ratio of the number of cells of NHDF to HUVEC is set to 0:100 (0% NHDF), 50:50 (50% NHDF), or 75:25 (75% NHDF). It was shown (in Figs 17 and 18) that, although three-dimensional tissue construct having capillaries can be formed with only HUVEC, the lumens can be more easily formed in the case where the ratio of the number of cells of NHDF to HUVEC is 25% to 75%.

### [Test Example 5: Effect of Amount of sCMF]

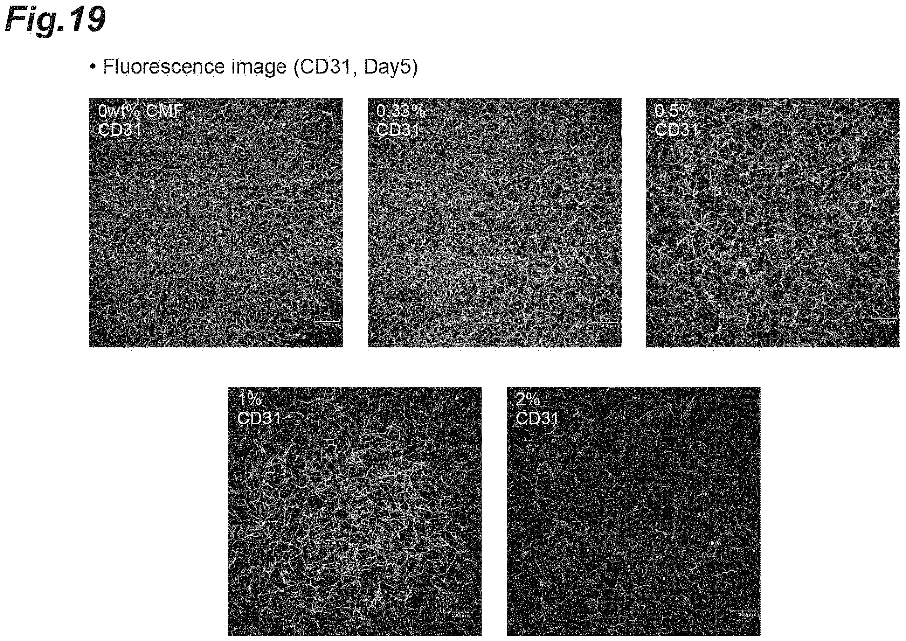

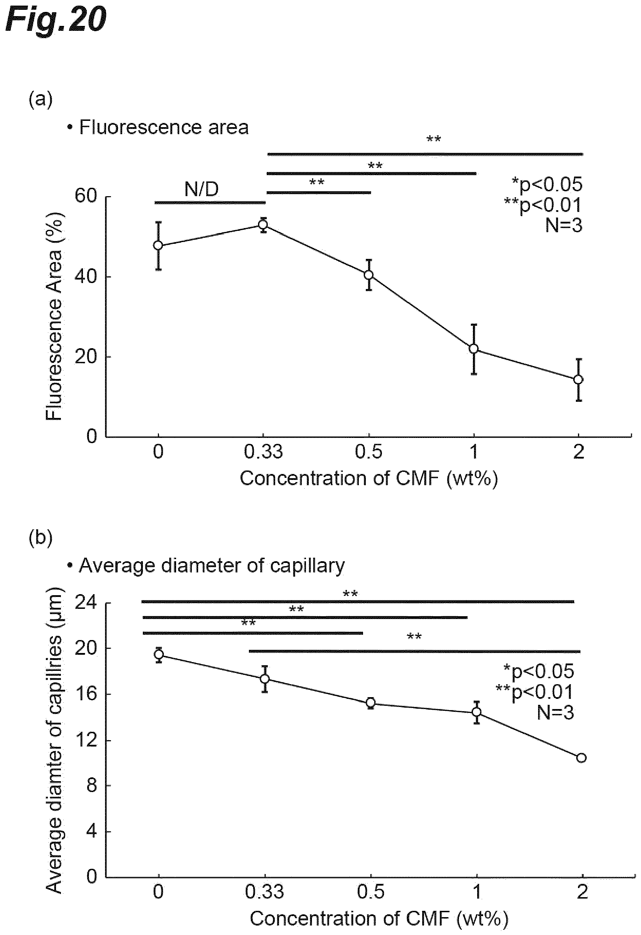

Three-dimensional tissue constructs were produced through the same method as described above using 0%, 0.33%, 0.5%, 1%, or 2% of sCMF with respect to the total amount of aqueous medium. Photographs showing observation results and measurement results of the diameter of the lumens are shown in Figs. 19 and 20. As shown in Figs. 19 and 20, it was shown that a blood vessel network can be formed in a case where sCMF is contained. In particular, in a case where the amount of sCMF was 0.22%, 0.5%, and 1%, a dense blood vessel network was formed.

### [Test Example 6: Effect of Amount of Fibrin]

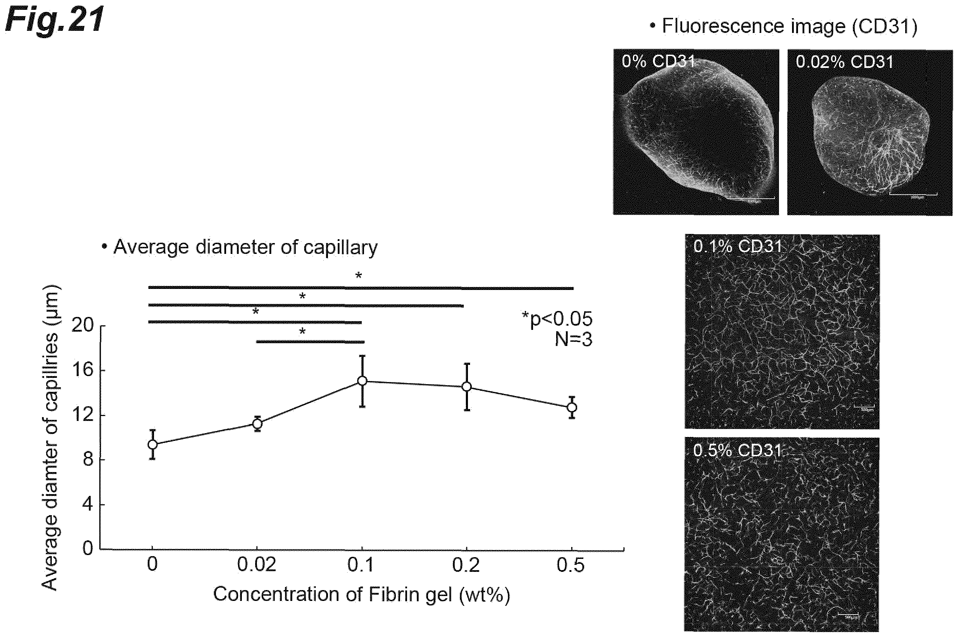

Three-dimensional tissue constructs were produced through the same method as described above using 0%, 0.02%, 0.5%, 0.1%, or 0.5% of fibrin with respect to the total amount of aqueous medium. As shown in Fig. 21, in a case where fibrin was not contained, an end portion of the three-dimensional tissue construct had strong fluorescence and formation of a blood vessel network was insufficient. On the other hand, in a case where fibrin was contained, a blood vessel network was sufficiently formed in a wide interior region.

### <Test Example 7: Evaluation of Stability of Three-Dimensional Tissue Construct>

A test solution for forming a construct which contains fibrinogen, thrombin, cells (NHDF:HUVEC=2:1), an aqueous medium DMEM, and sCMF was prepared. The amount of fibrinogen with respect to the test solution was set to 20 mg/mL, and the amount of thrombin with respect to the test solution was set to 30 unit/mL. Regarding the cells, the total number of cells was set to 5 × 10⁶ cells/mL. The amount of sCMF was set to 0.5 mass% with respect to the total mass of sCMF and the cells. In addition, a comparative test solution was produced similarly to the test solution for forming a construct except that no sCMF was used. 30 µL of each test solution was placed in the form of a liquid droplet. The point in time when a liquid droplet of each test solution was placed was set as a reference time (0 minutes), and an aqueous medium was placed on the liquid droplet of each test solution at a point in time when 30 minutes had elapsed. At a point in time when 90 minutes had elapsed from the reference time, 3 mL of a DMEM medium was added thereto, and the cells were cultured for 10 days under the condition of 37°C.

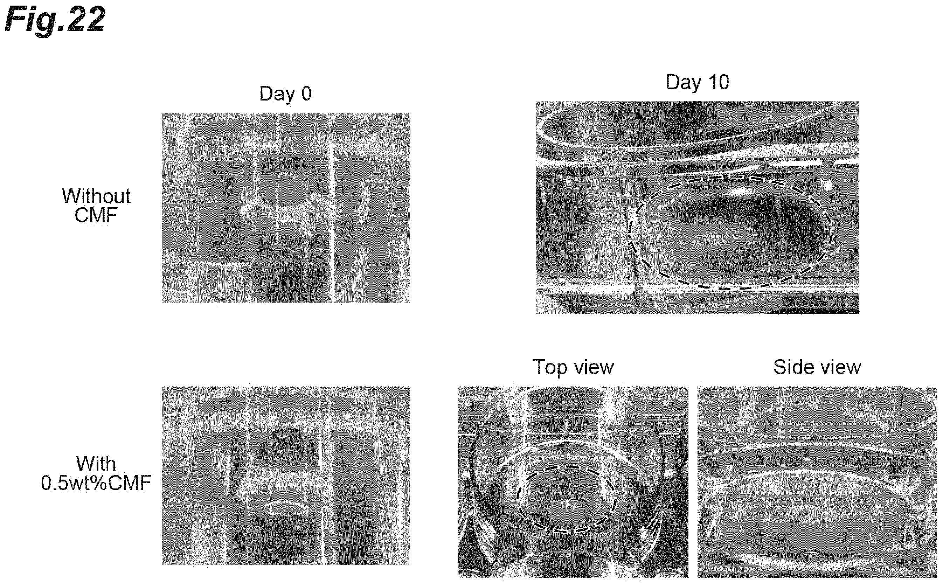

It was shown (in Fig. 22) that, in a case where sCMF was used, the shape of a three-dimensional tissue construct was sufficiently maintained before and after the lapse of 10 days.

### [Test Example 8: Evaluation of Behavior of Cell Population]

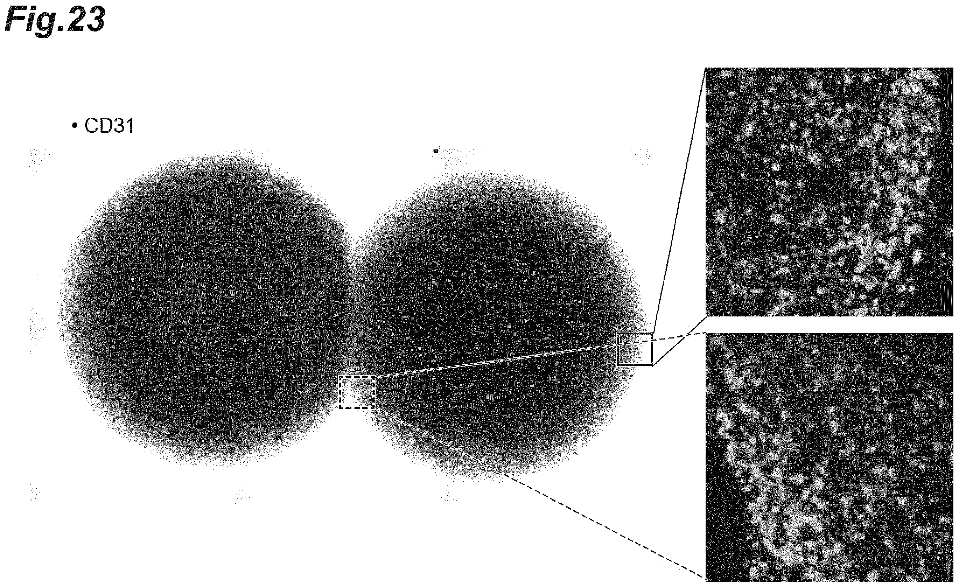

A test solution for forming a construct produced in the same manner as in Test Example 2 except that 1.0 × 10⁵ HUVEC cells (Lonza, C2517A) were used was seeded in a glass bottom dish. Three types of gels (no cells, 1.0 × 10⁵ NHDF cells (Lonza, CC-2509), and 3 ng of VEGF and 1.0 × 10⁵ NHDF cells) produced in the same manner as in Test Example 2 except that there were not cells or the types and the amount of cells contained varied were seeded after incubating for 10 minutes at 37°C so as to be adjacent to each other, and the gells were incubated at 37°C for 2 hours. Thereafter, 3 mL of a DMEM (50%)/EBM-2 (50%, NACALAI TESQUE, INC., Lonza, CC-3202) medium was added thereto and cultured for 1 week. Immunostaining was performed using CD31 (Dako, M0823) and Alexa647 (Invitrogen, A21235), and fluorescence observation was performed using Confocal Quantitative Image Cytometer CQ (YOKOGAWA) to evaluate the formation of a capillary network. The results are shown in Figs. 23 to 25.

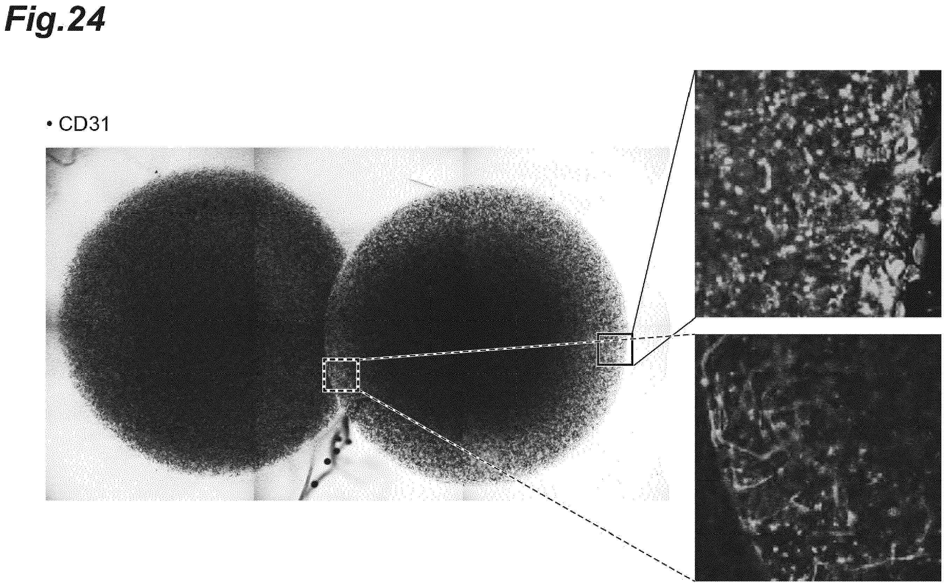

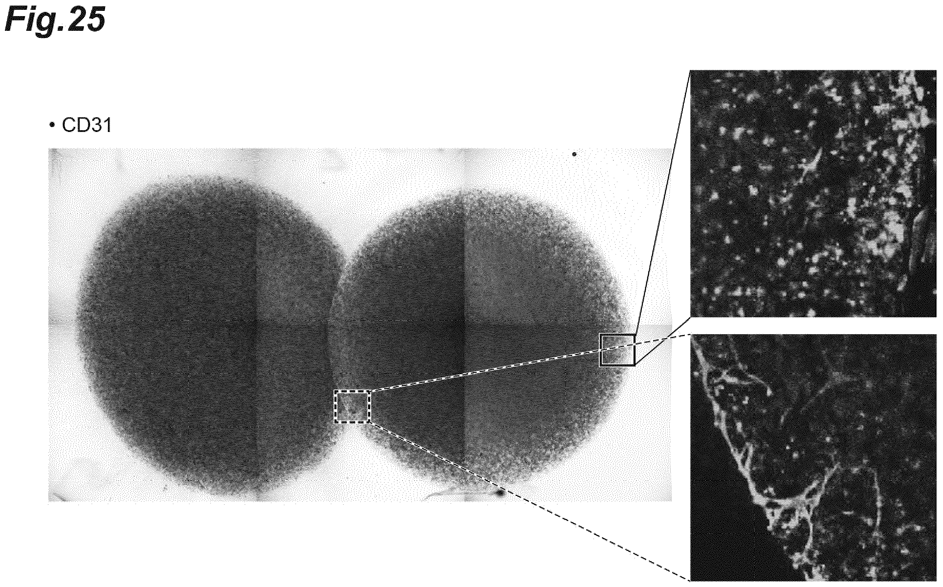

It was confirmed that angiogenesis was induced near the interfaces in Figs. 24 and 25.

## Claims

1. A method for producing a three-dimensional tissue construct, the method comprising:
a culture step of culturing cells in a culture liquid comprising fragmented extracellular matrix components, fibrin, and an aqueous medium.

2. The method for producing a three-dimensional tissue construct according to claim 1, the method further comprising:
a step of mixing fibrinogen with thrombin before the culture step.

3. The method for producing a three-dimensional tissue construct according to claim 1 or 2,
wherein at least some of the fragmented extracellular matrix components is cross-linked.

4. The method for producing a three-dimensional tissue construct according to any one of claims 1 to 3,
wherein an average length of the fragmented extracellular matrix components is 100 nm to 200 µm.

5. The method for producing a three-dimensional tissue construct according to any one of claims 1 to 4,
wherein the cells comprise extracellular matrix-producing cells.

6. The method for producing a three-dimensional tissue construct according to any one of claims 1 to 5,
wherein the cells further comprise one kind or plural kinds of cells selected from the group consisting of vascular endothelial cells, cancer cells, cardiomyocytes, smooth muscle cells, fibroblasts, and epithelial cells.

7. The method for producing a three-dimensional tissue construct according to any one of claims 1 to 6,
wherein the cells comprise at least vascular endothelial cells and fibroblasts.

8. The method for producing a three-dimensional tissue construct according to claim 7,
wherein a content of the fibroblasts is greater than or equal to 25% based on the number of whole cells.

9. The method for producing a three-dimensional tissue construct according to any one of claims 1 to 8,
wherein a content of the fragmented extracellular matrix components is 0.33 mass% to 90 mass% based on the total mass of the fragmented extracellular matrix components and the cells.

10. The method for producing a three-dimensional tissue construct according to any one of claims 1 to 9,
wherein a content of the fragmented extracellular matrix components is 0.5 mass% to 90 mass% based on the total mass of the fragmented extracellular matrix components and the cells.

11. The method for producing a three-dimensional tissue construct according to any one of claims 1 to 10,
wherein the culture liquid is placed while being moved in a substantially horizontal direction.

12. A method for producing a cell-containing composition, the method comprising:
a step of arranging a first liquid droplet comprising at least fragmented extracellular matrix components, fibrin, an aqueous medium, and cells and a second liquid droplet comprising at least an aqueous medium so that the liquid droplets come into contact with each other.

13. The method for producing a cell-containing composition according to claim 12,
wherein the second liquid droplet is placed on the first liquid droplet.

14. A three-dimensional tissue construct comprising:
fragmented extracellular matrix components;
fibrin; and
cells.
